# EUROPEAN PATENT SPECIFICATION

(11) **EP 4 452 225 B1**
(45) Date of publication and mention of the grant of the patent: **24.06.2026**
(21) Application number: 22843882.6
(22) Date of filing: 20.12.2022
(51) Int. Cl.: A61K 9/00, A61L 27/20

(54) **NOVEL BIOCOMPATIBLE SUBSTITUTES OF THE VITREOUS HUMOR**
NEUE BIOKOMPATIBLE ERSATZPRODUKTE DES GLASKÖRPERS
NOUVEAUX SUBSTITUTS BIOCOMPATIBLES DE L'HUMEUR VITRÉE

(30) Priority: 22.12.2021 IT 202100032111
(43) Date of publication of application: 30.10.2024
(73) Proprietor: Fidia Farmaceutici S.p.A., 35031 Abano Terme (PD) (IT)
(72) Inventor: GUARISE, Cristian, 35031 ABANO TERME (PD) (IT); RUSCIANO, Dario, 35031 ABANO TERME (PD) (IT)
(74) Representative: Bianchetti & Minoja with Trevisan & Cuonzo IPS SRL
(86) International application number: PCT/IB2022/062536
(87) International publication number: WO 2023/119152

(56) References cited:
- US-A- 4 716 154
- US-A1- 2016 067 274
- US-B2- 7 683 038

## Description

### Field of the invention

Object of the present invention are compositions based on hexadecyl amide of hyaluronic acid, having an amidation degree comprised in the range 1-3%, prepared starting from hyaluronic acid of molecular weight (MW) comprised in the range 500-730 kDa (HYADD^{®}-4), for use in the surgical treatment of the anterior and posterior segments of the eye, preferably in the surgical treatment of the posterior segment of the eye, including the vitreous chamber, even more preferably as vitreous humor substitute following vitrectomy.

### Background art

The human eye wall consists, from the outside to the inside, of three concentric tunics: fibrous tunic, vascular tunic or uvea, and nervous tunic.

The fibrous tunic performs primarily a structural and protective function, and it is composed by sclera and cornea. The sclera is the white opaque portion composed by collagen and elastic fibers covering externally about five-sixths of the eyeball in the posterior part, while the cornea is the positioned-anteriorly multilayered transparent structure covering a portion of about one-sixth.

The trophic and vascular functions are performed from the vascular tunic or uvea, comprising iris, ciliary body, and choroid. The iris is the anterior segment of the uvea, characterized by a thin ring structure, whose characteristic pigmentation defines the color of the eyes and whose central hole is called the pupil. Instead, the ciliary body extends from the iris to the choroid and comprises the ciliary muscle, main responsible of focusing. In fact, the ciliary muscle, contracting and relaxing, is able to determine a variation of the curvature, and therefore of the dioptric power, of the natural lens of the eye connected to it, the crystalline. Instead, the function of support and maintenance of the crystalline position is performed by a taut fiber system between the crystalline itself and the ciliary body, termed ciliary zonule or crystalline suspensor apparatus. Instead, the choroid is a structure extremely rich in blood vessels and capillaries, occupying the posterior portion of the uvea constituting a thin layer between the sclera and the retina. The border area between the ciliary body and the choroid is termed *ora serrata.*

Finally, the retina or nervous tunic is the component of the eye sensitive to light, and consists of different layers of neurons interconnected by synapses and a pigmented epithelium providing trophic and functional support. The retina adheres internally to the posterior third of the eyeball, and its positioning is essential to ensure its function, allowing to collect the light stimuli penetrating the eyeball through the pupil (Gray H. & Lewis W. H., 1918; Anatomy of the human body, 20th ed. Philadelphia: Lea & Febiger).

In ophthalmology, the separation of the eye in two different portions: the anterior segment and the posterior segment assumes a considerable importance, both from a diagnostic and surgical point of view.

The anterior segment comprises the cornea, iris, and pupil, crystalline with the structures sustaining it, and ciliary body. These structures identify two cavities: the anterior chamber, delimited from the posterior side of the cornea and the anterior side of the iris, and the posterior chamber, comprised between the iris and the formations binding the crystalline to the ciliary body. These cavities, communicating through the pupil, are occupied by the aqueous humor, a liquid formed by secretion from the ciliary body and made predominantly of water, amino acids, and electrolytes. Such liquid acts as a refractive medium, providing nutrients to the cornea and crystalline and contributing to keep the intraocular pressure.

The anterior segment is the eye portion more exposed to traumatic lesions, but also several pathologies which can affect it are known, such as allergies or cornea infections, dry-eye syndrome, and the keratoconus, *i.e*., a cornea degenerative pathology leading to a progressive deformation thereof. The cataract is one of the best known pathologies affecting the anterior segment of the eye. To date, the cataract, characterized by the crystalline opacification, is treated successfully by a surgical treatment providing for the replacement of the natural crystalline with an artificial intraocular lens. The cataract is one of the most frequent ocular pathologies, and the surgeries directed to the resolution of this pathology are constantly increasing (Moshirfar M et al., StatPearls 2021 [PMID: 32644679]). In the case of surgeries of the anterior segment of the eye, both related to the cataract treatment and to other pathology, it is required the use of a viscoelastic material providing a physical barrier or cushion between the surgical instruments and the tissues, and able to keep wide open the cavity of the anterior chamber and the capsular bag during the surgery (Maltese et al., Biomaterials 2006, 27:5134-42).

The posterior segment of the eye comprises the posterior two-thirds of the eyeball and the cavity comprised between the posterior side of the crystalline and the retina, known as vitreous chamber. Such chamber is filled by a clear gel termed vitreous humor, consisting principally of water, sugars, salts, hyaluronic acid, and collagen. The vitreous humor performs principally a structural function, keeping the spherical shape of the eye, but it is involved also in the adjustment of the intraocular pressure and, consequently, of the maintenance in place of the retina (Alovisi et al., *Journal of Ophthalmology* 2017, 2017:3172138).

Besides severe traumas interesting the whole eyeball and infections of the internal part of the eye, the posterior segment of the eye may be interested by a great number of pathologies, including:
- diabetic retinopathy, a pathology arising due to the damages which diabetes mellitus causes to the small blood vessels and neurons constituting the retina, and representing one of the major leading causes of blindness in developed countries;
- retinal detachment, whereby the retina detaches from the inside of the eyeball to which adheres normally, losing its own placement and thus determining the vision loss;
- macular hole or formation of an epiretinal membrane, also known as macular pucker in the central portion of the retina (macula);
- vitreous hemorrhage, *i.e.,* an intraocular bleeding, generally secondary to other pathologies, such as diabetic retinopathy or retinal detachment;
- presence of floaters, or thickening, in the vitreous humor (vitreous floaters);
- complications of surgeries of the anterior segment of the eye, such as following cataract surgery, impacting the posterior segment.

In all the above mentioned situations, it may be required to intervene surgically on the posterior segment of the eye. The most frequent surgical procedure involving the posterior segment of the eye is vitrectomy, *i.e.,* the removal of the vitreous humor and its total or partial replacement with an analogue artificial medium, which can be performed alone or in combination with other procedures aimed to, for example, reposition or repair the retina.

Given the multiplicity of the situations, and therefore the high frequency, with which vitrectomy turns out to be necessary, developing artificial media suitable to act as substitutes of the vitreous humor is of great interest. Among the desirable characteristics for a substitute of the vitreous humor, the following can be highlighted herein:
- it should show rheologic characteristics and refraction index similar to the natural vitreous humor;
- it should be able to keep the normal intraocular pressure;
- it should be easy to handle;
- it should be biocompatible and not toxic;
- it should be stable in the vitreous chamber, so as to remain *in loco* for all the required time.

To date, the only substance accepted as a long term substitute of the vitreous humor is the silicone oil. The silicone oil is a liquid siloxane polymerized with organic side chains. It is a hydrophobic polymer with a refraction index similar to the natural vitreous and density slightly lower than the water density, which, although suitable for use as substitute of the vitreous humor, shows some great disadvantages, including, for example:
- it should be removed after about 3 months from the surgery or in the case of occurrence of side effects, such as the oil emulsion or secondary glaucoma, thus requiring a second surgery which, in some cases, can even result in a new retinal damage;
- the buffering ability towards the lower part of the retina *(i.e.,* of the retinal portion placed in the eye lower quadrant) is reduced due to the low specific density of the normal silicone oil.

In order to favor the injury repair at the lower part of the retina, a mixture of silicone oil and partially fluorinated octane, which is referred to as heavy silicone oil (HSO) having a higher specific weight, was used but an intraocular pressure increase was observed beyond the possible onset of a series of complications including cataract, inflammation, and oil emulsion (Alovisi et al., Journal of Ophthalmology 2017, 2017:3172138).

Among the possible alternatives currently under study, the candidates for the replacement of the vitreous humor based on chemically-modified or derivatized artificial or natural polymers are of great interest. The hyaluronic acid is one of the polymers which are most suitable for the purpose.

The hyaluronic acid (HA) is a heteropolysaccharide consisting of alternate residues of D-glucuronic acid and N-acetyl-D-glucosamine. It is a linear chain natural polymer with molecular weight ranging from 50,000 to 13 x 10⁶ Da, according to the source from which it is obtained and the used preparation methods. It is naturally present in pericellular gels, the fundamental substance of the connective tissue of the vertebrate organisms (of which it is one of the main components), the synovial fluid of joints and the umbilical cord. Also, HA is one of the main components of the natural vitreous humor. Therefore, HA plays an important role in the biological organism, mostly as mechanical support of the cells of multiple tissues such as the skin, tendons, muscles, and cartilage.

Further, it is known how HA, through its membrane receptor CD44, modulates several and different processes related to cell physiology and biology such as the proliferation, migration, cellular differentiation, and angiogenesis, and also performs other functions such as tissue hydration and joint lubrification.

Among the products containing native HA used in ophthalmic surgery, the main is Healon^{®}, based on high molecular weight HA (4 x 10⁶ Da). Anyway, to date Healon^{®} is indicated only for use in the surgery of the anterior segment of the eye, but it shows not optimal rheologic properties and some studies wherein it was tested as substitute of the vitreous humor highlighted a significative increase of the intraocular pressure and the onset of side effects in treated patients (Koster et al., Documenta Ophthalmologica 1986, 64:13-17; Folk et al., Ophthalmic Surgery 1986, 17:299-306; Vatne et al., Acta Ophthalmol Copenh 1986, 64:169-72). Based on these and numerous other evidences, the substitutes of the vitreous humor based on native HA have not found wide use in the surgical practice.

Numerous HA derivatives are described:
- HYAFF^{®}: HA esters (EP216453 B1);
- HYADD^{®}: HA amides (EP1095064 B1);
- O-sulphate HA derivatives up to 4^{th} sulphation degree (EP702699 B1);
- ACP^{®}: HA internal esters (EP341745 B1);
- Hylan G-F20: HA cross-linked with formaldehyde and divinyl-sulphone (US4713448);
- HA deacetylates: derive from the deacetylation of the moiety N-acetylglucosamine (EP1313772 B1);
- HYOXX^{®}: per-carboxylated HA derivatives (patent application EP1339753);
- HA cross-linked with BDDE (EP0839159 B1).
- percarboxylated hyaluronic acid (patent US 7683038B2).

For some of the aforementioned HA derivatives, the use in a generic context of ophthalmic surgery was supposed, but no derivative was made eligible as product for use in ophthalmic surgery. Further, studies ensuring its non-toxicity and the possessing of physiological characteristics desirable for the purpose are not available.

In literature, numerous possible substitutes of the vitreous humor based on chemically-modified HA, mostly by cross-linking, are described. Such for example in patent US 4716154A.

The use of a hydrogel based on diidrazide of oxidized HA/adipic acid (Oxi-HA/AADH) as substitute of the vitreous humor, having a storage modulus G' comprised between 100 and 150 Pa, was described (Su et al., Journal of Biomaterials Science 2011, 22:1777-97). For the same use, a hydrogel based on thiolate, cross-linked by induction of the formation of disulphide bridges HA (cross-linked HA-thiol), characterized by a quite high storage modulus G,' equal to 150.4 Pa was described also (Schnichels et al., PloS 2017, 12:e0172895). The rheologic properties of these products differ significantly from those of the natural vitreous humor, having a storage modulus G' comprised between 1.86 and 6.35 Pa (Silva et al., Rheological Acta 2017, 56:377-86).

Patent US 2016/067274A1 discloses the use of hyaluronic acid hexadecylamide for the replacement of nucleus pulposus.

As possible substitute of the vitreous humor, a hydrogel based on HA cross-linked with 1,4-butanediol diglycidyl ether (BDDE), trade name Healaflow^{®} (US20100069938), was also tested. The toxicity of BDDE is known, making potentially dangerous the use of products wherein residues of this substance could be present (Jeong et al., Toxicology in Vitro 2021, 70:105034). Further, preclinical studies wherein Healaflow^{®} was tested as substitute of the vitreous humor highlighted the medium viscosity loss in the post-operative period and a slight increase of the intraocular pressure (IOP), potentially dangerous in particular for the reparation of retinal damages (Barth et al., Graefe's Archive for Clinical and Experimental Ophthalmology 2016, 254:697-703).

### Description of the invention

The Applicant found surprisingly that compositions based on hexadecyl amide of hyaluronic acid, having a molecular weight (MW) equal to (or comprised between) 500-730 kDa, having a amidation (derivatization) degree comprised in the range 1-3%, are particularly suitable for use in the surgical treatment of the anterior and posterior segments of the eye, preferably in the surgical treatment of the posterior segment of the eye, including the vitreous chamber, even more preferably as substitute of the vitreous humor following vitrectomy.

In scope of the present invention, the above-described hexadecyl amide of hyaluronic acid with MW equal to 500-730 kDa and having a amidation (derivatization ) degree comprised in the range 1-3%, does not have any other chemical modification (or chemical bond) of/in the hydroxy and/or carboxy groups thereof as well as in any other functional group; consequently, object of the inventions are compositions comprising hexadecyl amide of hyaluronic acid having an amidation degree comprised in the range 1-3%, wherein the hexadecyl amide of hyaluronic acid is obtained by derivatization of hyaluronic acid with MW comprised between 500 and 730 kDa, for use in the surgical treatment of the eye.

The preparation of the hexadecyl amide having a 1-3% average amidation (derivatization) degree, starting from HA having molecular weight (MW) equal to 500-730 kDa, is described in EP2988792; such a derivative, hereinafter referred to as **HYADD^{®}-4,** is commercially available at a concentration of 8 mg/ml in saline solution with the trademark Hymovis^{®} and has long been used exclusively for the intra-articular viscosupplementation therapy of osteoarthrosis.

The following reported data demonstrate that HYADD^{®}-4 is not toxic in such surgery and has rheological characteristics nearer to the natural vitreous humor. Further, HYADD^{®}-4 is extrudable easily through a syringe, therefore being easily to handle and inject in the vitreous chamber.

According to the invention, HYADD^{®}-4 was used as it is (HYADD^{®}-4) or in a depolymerized form obtained by thermic treatment at 121 °C for 25 minutes or more. Preferably, depolymerized HYADD^{®}-4 is obtained by thermic treatment at 121 °C for 120 minutes.

The following experiments, carried out on animals subjected to retinal damage and subsequent vitrectomy with complete replacement of the vitreous humor, allowed to highlight that:
- HYADD^{®}-4 does not determine any anomaly in the post-operative IOP, therefore being ideal to favor the keeping in position and healing of the retina;
- HYADD^{®}-4 is biodegradable, in fact after 30 days from the complete replacement of the vitreous is present in percentage amounts reduced with respect to the initial 100%, and therefore does not require a second surgery for removal from the vitreous chamber;
- HYADD^{®}-4 is stable in the vitreous chamber, and its stability can be modulated through depolymerization, as highlighted from the higher stability observed for HYADD^{®}-4 as it is (not depolymerized) with respect to depolymerized HYADD^{®}-4;
- HYADD^{®}-4 promotes a better recovery from the retinal damage with respect to the silicone oil, currently in use in the clinical practice, as demonstrated from the higher protein levels of the retinal functionality biomarker transthyretin (TTR) and the reduction of the intra-vitreal hemoglobin levels, a blooding index;
- HYADD^{®}-4 determines a better post-operative course with respect to the silicone oil following damage located in the lower quadrant of the retina, with reduced presence of ocular inflammation signs such as hyperemia or redness, chemosis, and discharge.

Based on these evidences, HYADD^{®}-4, as it is or depolymerized, emerges as an excellent viscoelastic medium for the ophthalmic surgery, particularly suitable as substitute of the vitreous humor.

### Detailed description of the invention

The invention relates to compositions based on hexadecyl amide of hyaluronic acid, having an amidation degree comprised in the range 1-3%, prepared starting from hyaluronic acid of molecular weight (MW) comprised in the range 500-730 kDa (HYADD^{®}-4), for use in the surgical treatment of the eye, preferably in the surgical treatment of the posterior segment of the eye, including the vitreous chamber. In particular, the use of the compositions of the invention as biocompatible substitutes of the vitreous humor following vitrectomy is particularly convenient.

The hyaluronic acid (HA) can have a molecular weight from 50,000 to 13 x 10⁶ Da. The starting HA used for the synthesis of the HA hexadecyl amide has a MW comprised between 500 and 730 kDa. For MW is meant the weight average molecular weight calculated with the "intrinsic viscosity" method (Terbojevich et al., Carbohydr Res, 1986, 149:363-377).

According to the invention, HA deriving from any source, for example, from cockscombs (EP138572), fermentation (from *Streptocuccus equi* or *zooepidemicus,* EP0716688), or biosynthesis (from *Bacillus,* EP2614088, EP2614087) can be used. The HA used in the present invention can also be purified according to different techniques (EP3491027, EP3655138). Preferably, the starting purified HA as described in EP3491027.

The HA derivative finding application according to the invention is the hexadecyl amide of hyaluronic acid with derivatization or amidation degree comprised in the range 1-3%, prepared starting from hyaluronic acid of molecular weight (MW) comprised in the range 500-730 kDa and does not have any other chemical modification (or chemical bond) of/in the hydroxy and/or carboxy groups thereof as well as in any other functional group.

The derivatization (or amidation) degree of HYADD^{®}-4 is measured by HPLC after amide hydrolysis and subsequent conjugation of the hexadecyl amine released with a fluorophore substance. The hexadecyl amide of hyaluronic acid with derivatization (or amidation) degree comprised in the range 1-3% and prepared starting from hyaluronic acid of molecular weight (MW) comprised in the range 500-730 kDa, can be prepared as known, preferably as described in EP2988792.

The hexadecyl amide of hyaluronic acid can be used as it is or depolymerized. For hexadecyl amide of hyaluronic acid (or HYADD^{®}-4) as it is it is meant the hexadecyl amide of hyaluronic acid, having a derivatization or amidation degree measured by HPLC comprised in the range 1-3%, prepared starting from hyaluronic acid of molecular weight (MW) comprised in the range 500-730 kDa, obtained according to known methods and preferably as described in EP2988792. For depolymerized hexadecyl amide of hyaluronic acid (or HYADD^{®}-4) it is meant the hexadecyl amide of hyaluronic acid (or HYADD^{®}-4) subjected to thermic treatment for at least 25 minutes at 121 °C. Preferably, the depolymerized hexadecyl amide of hyaluronic acid (or HYADD^{®}-4) is obtained subjecting the hexadecyl amide of hyaluronic acid (or HYADD^{®}-4) to a thermic treatment for at least 2 hours at 121 °C.

Starting from the hexadecyl amide of hyaluronic acid having a derivatization degree comprised in the range 1-3% (HYADD^{®}-4) as it is or depolymerized, a hydrogel at a concentration equal to or lower than 12 mg/mL can be formulated. Preferably, the concentration of HYADD^{®}-4 as it is or depolymerized in the hydrogel of the invention is equal to 8 mg/mL. HYADD^{®}-4 as it is or depolymerized can be dissolved in any suitable medium, such as physiological solution or saline phosphate buffer, preferably in saline phosphate buffer at pH 7.

The compositions comprising HYADD^{®}-4 as it is or depolymerized for use in the surgical treatment of the anterior and posterior segments of the eye can be possibly formulated adding pharmaceutical acceptable excipients.

According to the invention, HYADD^{®}-4 as it is or depolymerized is particularly suitable for use in the surgical treatment of the anterior and posterior segments of the eye, preferably in the surgical treatment of the posterior segment of the eye, including the vitreous chamber, even more preferably as substitute of the vitreous humor following vitrectomy. By way of example, HYADD^{®}-4 as it is or depolymerized can be used in the surgical treatment of the anterior segment of the eye, and in particular as viscoelastic medium in the cataract surgery. Further, HYADD^{®}-4 as it is or depolymerized is used in the surgical treatment of the posterior segment of the eye, as substitute of the vitreous humor following vitrectomy, wherein the vitrectomy may have become necessary for any reason. Some examples of pathologies for which performing a vitrectomy may be necessary and for which the use of HYADD^{®}-4 as it is or depolymerized can be advantageous, are:
- severe traumas interesting the whole eyeball;
- infection of the internal part of the eye;
- diabetic retinopathy;
- retinal detachment;
- macular hole or formation of an epiretinal membrane, also known as macular pucker in the central portion of the retina (macula);
- vitreous hemorrhage;
- presence of floaters, or thickening, in the vitreous humor (vitreous floaters);
- complications of surgeries of the anterior segment of the eye, such as following cataract surgery, impacting the posterior segment.

The following experiments, carried out on animals subjected to retinal damage and subsequent vitrectomy with complete replacement of the vitreous, allowed to highlight that:
- HYADD^{®}-4 does not determine any anomaly in the post-operative IOP, therefore being ideal to favor the keeping in position and healing of the retina;
- HYADD^{®}-4 is biodegradable, in fact after 30 days from the complete replacement of the vitreous humor is present in percentage amounts reduced with respect to the initial 100%, and therefore does not require a second surgery for removal from the vitreous chamber;
- HYADD^{®}-4 is stable in the vitreous chamber, and its stability can be modulated through depolymerization, as highlighted from the higher stability observed for HYADD^{®}-4 as it is (not depolymerized) with respect to depolymerized HYADD^{®}-4;
- HYADD^{®}-4 promotes a better recovery from the retinal damage with respect to the silicone oil, currently in use in the clinical practice, as demonstrated from the higher protein levels of the retinal functionality biomarker transthyretin (TTR) and the reduction of the intra-vitreal hemoglobin levels, a blooding index;
- HYADD^{®}-4 determines a better post-operative course with respect to the silicone oil following damage located in the lower quadrant of the retina, with reduced presence of ocular inflammation signs such as hyperemia or redness, chemosis, and discharge.

Based on these evidences, HYADD^{®}-4, as it is or depolymerized, emerges as an excellent viscoelastic medium for the ophthalmic surgery, particularly suitable as substitute of the vitreous humor.

To better illustrate the invention, the following examples are provided.

### EXAMPLE 1. Preparation of the hexadecyl amide of hyaluronic acid (HYADD^{®}-4) with different polymerization degrees.

The hexadecyl amide of hyaluronic acid with derivatization degree comprised in the range 1-3% (HYADD^{®}-4), prepared starting from HA with MW comprised between 500 and 730 kDa as described in EP2988792, was dissolved in PBS at pH 7 at the final concentration of 8 mg/mL. The depolymerization was obtained successively subjecting HYADD^{®}-4 in PBS at thermic treatment in autoclave, at 121 °C for:
- 25 minutes in the case of depolymerized HYADD^{®}-4;
- 120 minutes in the case of depolymerized HYADD^{®}-4.

### EXAMPLE 2. Rheological analysis of the hexadecyl amide of hyaluronic acid (HYADD^{®}-4), as it is and with different polymerization degrees.

### Materials and Methods

The following samples were subjected to rheological analysis:
- HYADD^{®}-4 as it is (a.i.i.), *i.e.,* hexadecyl amide of hyaluronic acid with derivatization degree comprised in the range 1-3%, prepared starting from HA with MW comprised between 500 and 730 kDa as described in EP2988792, and dissolved in PBS at pH 7 at the final concentration of 8 mg/mL;
- depolymerized HYADD^{®}-4 (25 min.), obtained by thermic treatment of HYADD^{®}-4 a.i.i. for 25 minutes at 121 °C, as described in EXAMPLE 1;
- depolymerized HYADD^{®}-4 (2 hours), obtained by thermic treatment of HYADD^{®}-4 a.i.i. for 2 hours at 121 °C, as described in EXAMPLE 1.

The rheological analysis was performed using a rheometer at 37 °C. The storage modulus G' was measured for all the samples at 1 Hz.

### Results and Conclusions

The rheological analysis allowed to detect the following G' values in Pascal (Pa) for the tested samples:

| **Rheological Analysis at 37 °C** | **G' at 0.1 Hz** |
|---|---|
| ***Sample*** | **(Pa)** |
| HYADD^{®}-4 a.i.i. | 28.2 |
| depolymerized HYADD^{®}-4 (25 min.) | 19.9 |
| depolymerized HYADD^{®}-4 (2 hours) | 1.3 |

These data strongly support the use of HYADD^{®}-4, as it is or depolymerized, as vitreous substitute. In fact, it is evident that depolymerized HYADD^{®}-4 (2 hours) has a G' completely overlapping to the G' reported for the vitreous humor (G'=1.86-6.35 Pa at 0.1 Hz and 37 °C; Silva et al., Rheological Acta 2017, 56:377-86). Instead, HYADD^{®}-4 as it is and depolymerized (25 minutes) have G' values quite comparable to those of the natural vitreous humor, but mostly lower than the G' (at 0.1 Hz and 37 °C) of the known candidates based on HA described as substitutes for the vitreous humor: in such conditions Oxi-HA/AADH has G' comprised between 100 and 150 Pa (Su et al., Journal of Biomaterials Science 2011, 22:1777-97), and cross-linked HA-thiol is characterized by a storage modulus G' equal to 150.4 Pa (Schnichels et al., PloS 2017, 12:e0172895), as below reported.

| **Rheological Analysis at 37 °C** | **G' at 0.1 Hz** |
|---|---|
| ***Sample*** | **(Pa)** |
| Natural vitreous humor | 1.86-6.35 |
| Oxi-HA/AADH | 100-150 |
| Cross-linked HA-thiol | 150.4 |

### EXAMPLE 3. In vivo evaluation of the stability and tolerability of the hexadecyl amide of hyaluronic acid (HYADD^{®}-4), as it is or depolymerized, as vitreous substitute after surgery of retinal damage and concomitant vitrectomy with complete replacement of vitreous.

### Materials and Methods

The scope of this first study is to verify the stability and tolerability in the vitreous chamber of the hexadecyl amide of hyaluronic acid (HYADD^{®}-4), as it is or depolymerized, in the retinal damage reparation. The study was carried out on 10 albino rabbits, for each one of them both the right and left eyes were treated, divided in the following experimental groups:
**1. *Silicone Oil*** group: 2 animals (4 eyes) treated with silicone oil;
**2. *HYADD^{®}-4*** group: 3 animals (6 eyes) treated with HYADD^{®}-4 as it is;
**3. *depolymerized HYADD^{®}-4*** group: 3 animals (6 eyes) treated with depolymerized HYADD^{®}-4, *i.e.,* HYADD^{®}-4 subjected to thermic treatment for 2 hours at 121 °C as for EXAMPLE 1;
**4. *CTRL*** group: 2 animals (4 eyes), not treated.

The albino rabbits belonging to the **Silicone Oil, HYADD^{®}-4** and **depolymerized HYADD^{®}-4** groups underwent surgery in both eyes, causing a retinal break by subretinal injection with sterile physiological solution and subsequent cut with scalpel. The margins of the retinal break were then treated with laser, and finally the vitreous substitute was inserted to obtain a filling of the vitreous chamber and a chorioretinal pessia of the break at one month from the surgery. The intraocular pressure in each treated eye was measured both before the surgery and at 1, 2, 3, 4, 5, 6, 7, 10, 15, 18, 20, 23, 25, 28 or 30 days after the surgery. After 30 days from the surgery, the animals were sacrificed and the vitreous humor of each eye was withdrawn for subsequent analyses.

The amount of HYADD^{®}-4 as it is or depolymerized present in each sample of vitreous humor was determined after amide hydrolysis and subsequent conjugation of the released hexadecyl amine with a fluorophore substance.

The transthyretin (TTR) and hemoglobin (Hb) values in the withdrawn samples of the vitreous humor were detected by ELISA assay, using proper commercial kits, and following the instructions provided by the manufacturer. Briefly, aliquots of constant volume of the different samples to be determined and of the standard for the quantification, *i.e.,* serial dilutions of a sample containing a known amount of the antigen of interest were distributed in the wells of a 96-well plate, pre-coated with antibodies able to recognize the antigen of interest. After a first incubation without reagents, a solution containing antibodies able to recognize the antigen of interest was added to each sample. Such antibodies are conjugated with an enzyme able to catalyze a colorimetric reaction and therefore, incubating the samples with the substrate of this colorimetric reaction, a colored product is produced in amounts directly proportional to the amount of the antigen of interest present in the sample (Leng et al., J Gerontol A Biol Sci Med Sci 2008, 63:879-84). The absorbance of each well at the end of the colorimetric reaction was detected using a spectrophotometer. The calculation of the exact antigen concentration in each sample is made possible applying the Lambert-Beer law, for which a linear relation exists between the concentration of a chromophore in solution and the absorbance of the solution itself (Oshina et al., Journal of Biomedical Optics 2021, 26:100901), and constructing a calibration line from the absorbance measurements of the standards for the quantification. In this way it is in fact possible to establish the conversion factor required to calculate the exact concentration of antigen corresponding to the absorbance value measured for each sample.

### Results and Conclusions

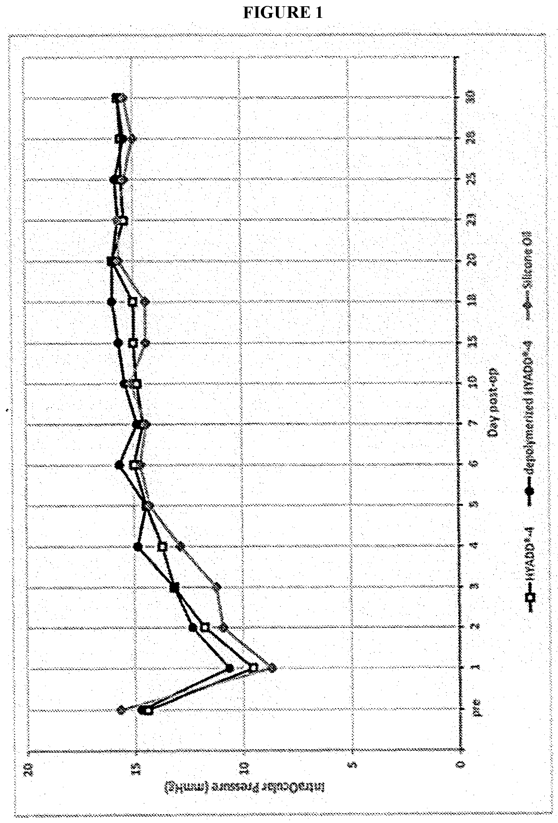

The monitoring of the intraocular pressure, whose results are shown in FIGURE 1, reveals an initial drop in all the tested conditions, likely due to the surgery, which is recovered within six days after the surgery. Subsequently, the IOP settles around values equal to the pre-surgery ones and remains almost constant until the end of the 30 days of monitoring. Since variations of the intraocular pressure are known interfering with the healing process following retinal damage (Alovisi et al., Journal of Ophthalmology 2017, 2017:3172138), the total absence of alterations in the IOP of the treated animals with HYADD^{®}-4, as it is or depolymerized, fully supports its use as substitute of the vitreous humor.

The detection of the residual amount of HYADD^{®}-4 present in the sample of the vitreous humor withdrawn from the treated rabbits highlighted how, at 30 days from the vitrectomy surgery, a 0.5% of depolymerized HYADD^{®}-4 (percentage on the total volume of the sample), and a 21.9% of HYADD^{®}-4 as it is (even in this case, understood as percentage on the total volume of the sample) persist. This result highlights how HYADD^{®}-4, as it is or depolymerized, is stable in the vitreous chamber, as demonstrated by the persistence in place at 30 days from the vitreous replacement. At the same time, HYADD^{®}-4 is also completely biodegradable, as demonstrated from the fact that its percentage amount with respect to the volume of the vitreous humor, likely equal to 100% at vitrectomy surgery with complete replacement of the vitreous humor, reduces drastically 30 days after the surgery, even reaching values next to zero in the case of depolymerized HYADD^{®}-4. Further, these data demonstrate how the depolymerization allows to modulate the residence time thereof in the vitreous chamber according to needs. In fact, HYADD^{®}-4, as it is, is able to remain in the vitreous chamber on the long term, representing about the 20% of the volume of the vitreous humor 30 days after the surgery, while depolymerized HYADD^{®}-4 persists for a medium to long term, being present in small amounts at 30 days from the surgery.

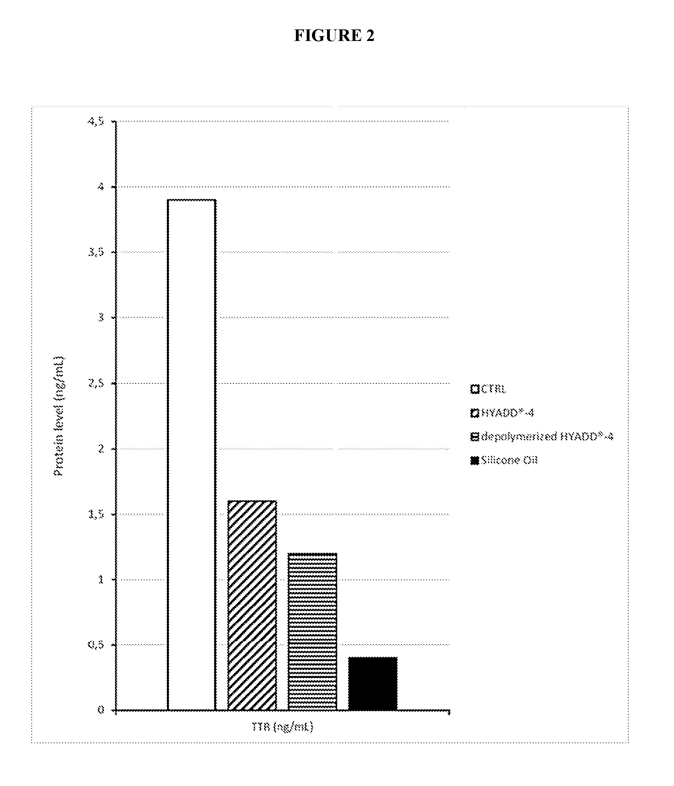

The transthyretin (TTR) levels are considered an optimal biomarker for the retinal functionality, being higher in conditions of higher visual acuity (Van Aken et al., Br J Ophthalmol 2009, 93:1539-45). As evident in FIGURE 2, the animals subjected to retinal damage receiving silicone oil as vitreous substitute show extremely low levels of TTR, averaged equal to 0.4 ng/mL, with respect to the 3.9 ng/mL of TTR detected in the vitreous samples withdrawn from not treated control animals. Surprisingly, in the vitreous samples withdrawn from animals treated with HYADD^{®}-4 as it is or depolymerized, much higher levels of TTR are detected, equal to 1.6 or 1.2 ng/mL, respectively, with respect to the silicone oil. Therefore HYADD^{®}-4, as it is or depolymerized, used as vitreous substitute stimulates an increase of the protein levels of TTR respectively equal to 4 or 3 times the one reached in the vitreous of animals treated with silicone oil, meaning that it promotes a better recovery of the retinal functionality with respect to the silicone oil, vitreous substitute presently in use in the surgical practice.

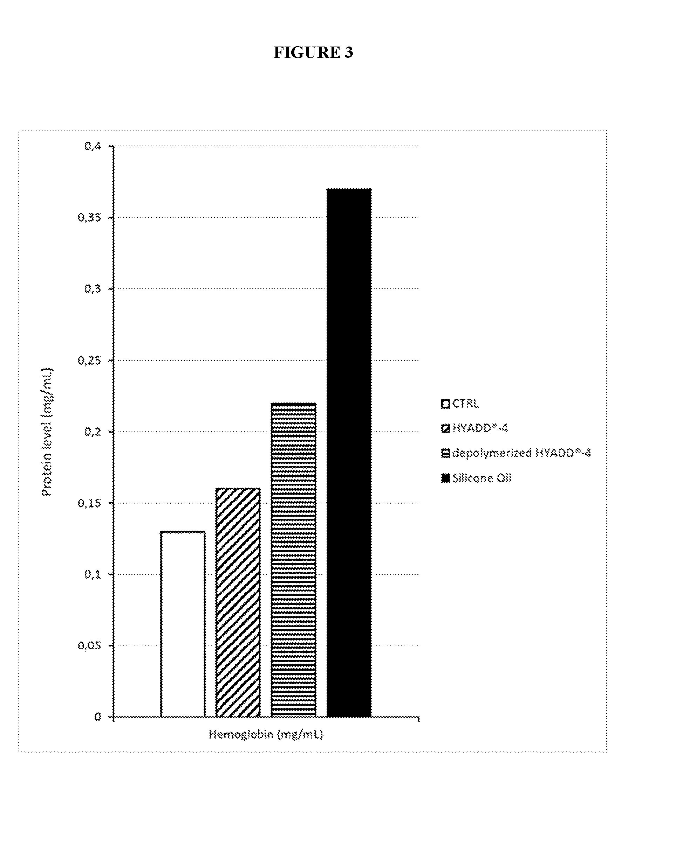

The hemoglobin is a protein present normally in red blood cells, where it plays the role of transporting oxygen through the blood stream, from the pulmonary alveoli where oxygen is absorbed to the peripheral tissues which use it for their own metabolism. In the vitreous humor the hemoglobin can be present physiologically in a very reduced amount, but in pathologic conditions it can increase considerably, such as in the presence of bleeding and/or intraocular damages and following to surgeries at ophthalmic level. As reported in FIGURE 3, the quantification of the protein levels of hemoglobin in the vitreous humor of the rabbits treated with silicone oil revealed how these are 3 times higher with respect to the hemoglobin levels in the vitreous humor of the not treated control rabbits (0.37 mg/mL in the case of treatment with silicone oil *vs* 0.13 mg/mL for the controls). The hemoglobin levels observed in the vitreous humor of animals treated with HYADD^{®}-4 as it is or depolymerized are respectively equal to 0.16 and 0.22 mg/mL, and therefore significantly lower than those observed in the case of the silicone oil, indicating a significantly better recover from the surgery.

In conclusion, HYADD^{®}-4 as it is or depolymerized is extremely suitable to act as vitreous substitute. In fact, the performed experimentation, and herein described, highlighted that:
- HYADD^{®}-4 does not determine any anomaly in the IOP, therefore being ideal to favor the keeping in position and healing of the retina;
- HYADD^{®}-4 is biodegradable, in fact after 30 days from the complete replacement of the vitreous humor is present in percentage amounts reduced with respect to the initial 100%, and therefore does not require a second surgery for removal from the vitreous chamber;
- HYADD^{®}-4 is stable in the vitreous chamber, and its stability can be modulated through depolymerization, as highlighted from the higher stability observed for HYADD^{®}-4 as it is (not depolymerized) with respect to depolymerized HYADD^{®}-4;
- HYADD^{®}-4 promotes a better recovery from the retinal damage with respect to the silicone oil, currently in use in the clinical practice, as demonstrated from the higher protein levels of TTR and the reduction of the hemoglobin levels.

### EXAMPLE 4. In vivo evaluation of the tolerability and efficacy of the hexadecyl amide of hyaluronic acid (HYADD^{®}-4), as it is or depolymerized, as vitreous substitute after surgery of retinal detachment induced by subretinal injection in the lower quadrant of the eye, with subsequent vitrectomy and complete replacement of the vitreous.

### Materials and Methods

The study was carried out on 9 New Zealand White (NZW) rabbits, for each of them both the right and the left eyes were treated, divided in the following experimental groups:
1. ***HYADD^{®}-4*** group: 3 animals (6 eyes) treated with HYADD^{®}-4 as it is;
2. ***Depolymerized HYADD^{®}-4*** group: 3 animals (6 eyes) treated with depolymerized HYADD^{®}-4, *i.e.,* HYADD^{®}-4 subjected to thermic treatment for 2 hours at 121 °C as for EXAMPLE 1;
**3. *Silicone Oil*** group: 3 animals (6 eyes) treated with silicone oil.

After being adequately anesthetized and following to accurate disinfection of the both eyes, the rabbits were subjected to surgical retinal detachment. The detachment interested a portion comprised between 1/4 and 1/3 of the whole retina, and it was obtained injecting 200 µL of Balanced Salt Solution (BSS) by a 38G needle in the lower quadrant of each eye. Subsequently, a retinotomy in the same area was performed, using a scalpel, and the retina was attached again by fluid-air change and laser treatment. Finally, immediately after the laser surgery, the treated eyes were subjected to the replacement of the vitreous humor with HYADD^{®}-4, depolymerized HYADD^{®}-4, or silicone oil according to the belonging group, using an instrument suitable for vitrectomy. After the surgical procedure, an antibiotic ointment was applied on the operated eyes and the rabbits were monitored until wake-up.

The retinal detachment state was monitored by Optical Coherence Tomography, OCT, allowing to visualize the eye fundus and therefore the retina itself, at 1, 7, 14 and 28 days from the surgery.

The tolerability was evaluated by eye macroscopic exam, oriented towards the detection of any obvious symptoms of irritation or ocular inflammation. The monitoring was performed one day before the surgery (baseline) and after 7 or 28 days from the surgery itself. Particular attention was paid to the following aspects:
- **Hyperemia,** *i.e.,* redness of the eye, known index of irritation and inflammation;
- **Chemosis,** or conjunctival edema, is a not-specific sign of eye irritation as well, showing as a protrusion of the bulbar conjunctiva with respect to the underlying tissues due to the formation of an edematous collection;
- **Ocular discharge,** which can be made of mucus, pus, or an excess of tears, connected to an irritation and/or inflammation status of the eye as well.

Each of these three aspects was quantified for each treated eye by the assignation of a score from 0 to 4, where 0 is understood as total absence of the symptom while 4 means that the symptom is present at the highest degree.

### Results and Conclusions

The monitoring of the retinal detachment by OCT allowed to detect the gradual healing of the retina in all the experimental groups. Within day 28, the majority of the retina detachments were in active healing, with few retinal sections detached. This evidence supports the efficacy of HYADD^{®}-4 as it is or depolymerized in adjuvating the healing of the retinal damage in the lower retinal quadrant.

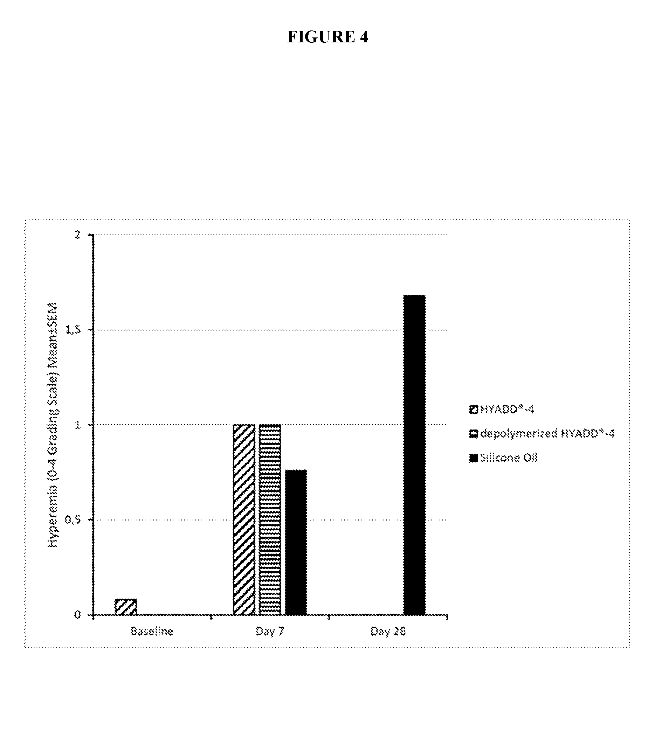

The observation of the macroscopic symptoms of irritation and inflammation of the eye, hyperemia, chemosis, and ocular discharge, was performed to evaluate the tolerability of HYADD^{®}-4 as it is or depolymerized in the vitreous chamber (Figures 4-6). One day before the surgery (baseline) all the animals did not show any sign of ocular irritation, obtaining an average score equal to or next to zero for each experimental group.

At 7 days from the surgery all the animals showed hyperemia. The symptom reduced, returning to the basal levels (equal to 0) at 28 days from the surgery, in the eyes treated with HYADD^{®}-4 as it is or depolymerized, while in the case of the silicone oil a significant worsening of the symptom was observed, with the obtaining of an average score higher than 1.5.

Chemosis and ocular discharge have occasionally shown up at 7 days from the surgery in the animals treated with HYADD^{®}-4 as it is or depolymerized, reaching an average score always less than 0.5, attesting negligible levels of these symptoms. After 28 days from the surgery, no animal treated with HYADD^{®}-4 as it is or depolymerized showed chemosis or ocular discharge symptoms (average score equal to 0 for each of both the symptoms), indicating the total absence of ocular irritation or inflammation signs. *Vice versa,* the animals treated with silicone oil showed relevant levels of chemosis at 7 days from the surgery, with an average score higher than 1.5, not even normalized after 28 days from the surgery, remaining at an average score equal to 1.5. Similarly, constant levels of ocular discharge with an average score higher than 0.5 were observed for the eyes treated with silicone oil both at 7 and 28 days from the surgery.

Overall, these data demonstrate that HYADD^{®}-4, as it is or depolymerized, determines a better post-operative course with respect to the silicone oil following damage located in the lower quadrant of the retina. In fact, the eyes treated with silicone oil show irritation/inflammation signs, both at 7 and 28 days from the surgery, at not negligible levels (as demonstrated by the average scores always higher than 0.5). *Vice versa,* the eyes treated with HYADD^{®}-4, as it is or depolymerized, do not show any symptom of ocular irritation or inflammation at 28 days (average scores equal to 0 both for hyperemia and for chemosis and ocular discharge).

### Brief description of the Figures

**FIG. 1****.** Monitoring of the intraocular pressure (IntraOcular Pressure, IOP) measured before (pre) and at different time ranges (1, 2, 3, 4, 5, 6, 7, 10, 15, 18, 20, 23, 25, 28 or 30 days) after the surgery of retinal damage with concomitant injection of vitreous substitute selected from: silicone oil (Silicone oil), hexadecyl amide of hyaluronic acid (HYADD^{®}-4), or hexadecyl amide of hyaluronic acid depolymerized by heat treatment (depolymerized HYADD^{®}-4).
**FIG. 2****.** Transthyretin levels (TTR), detected by ELISA assay, in the vitreous humor of not treated rabbits (CTRL) or after 30 days from retinal damage with concomitant injection of vitreous substitute selected from: silicone oil (Silicone oil), hexadecyl amide of hyaluronic acid as it is (HYADD^{®}-4), or hexadecyl amide of hyaluronic acid depolymerized by heat treatment (depolymerized HYADD^{®}-4).
**FIG. 3****.** Hemoglobin levels (Hemoglobin, Hb), detected by ELISA assay, in the vitreous humor of not treated rabbits (CTRL) or after 30 days from retinal damage with concomitant injection of vitreous substitute selected from: silicone oil (Silicone oil), hexadecyl amide of hyaluronic acid (HYADD^{®}-4), or hexadecyl amide of hyaluronic acid depolymerized by heat treatment (depolymerized HYADD^{®}-4).
**FIG. 4****.** Evaluation of the ocular hyperemia showed from rabbits in normal conditions (Baseline) and after 7 (Day 7) or 28 (Day 28) days from the surgery of retinal detachment induced by subretinal injection in the lower quadrant of the eye, with subsequent vitrectomy and complete replacement of the vitreous with silicone oil (Silicone oil), hexadecyl amide of hyaluronic acid (HYADD^{®}-4), or hexadecyl amide of hyaluronic acid depolymerized by heat treatment (depolymerized HYADD^{®}-4).

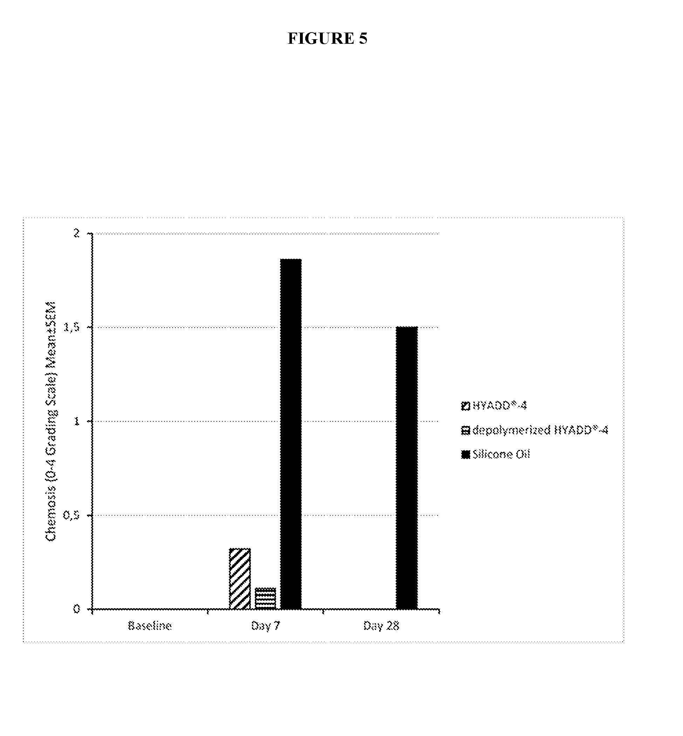

**FIG. 5****.** Evaluation of the chemosis showed from rabbits in normal conditions (Baseline) and after 7 (Day 7) or 28 (Day 28) days from the surgery of retinal detachment induced by subretinal injection in the lower quadrant of the eye, with subsequent vitrectomy and complete replacement of the vitreous with silicone oil (Silicone oil), hexadecyl amide of hyaluronic acid (HYADD^{®}-4), or hexadecyl amide of hyaluronic acid depolymerized by heat treatment (depolymerized HYADD^{®}-4).

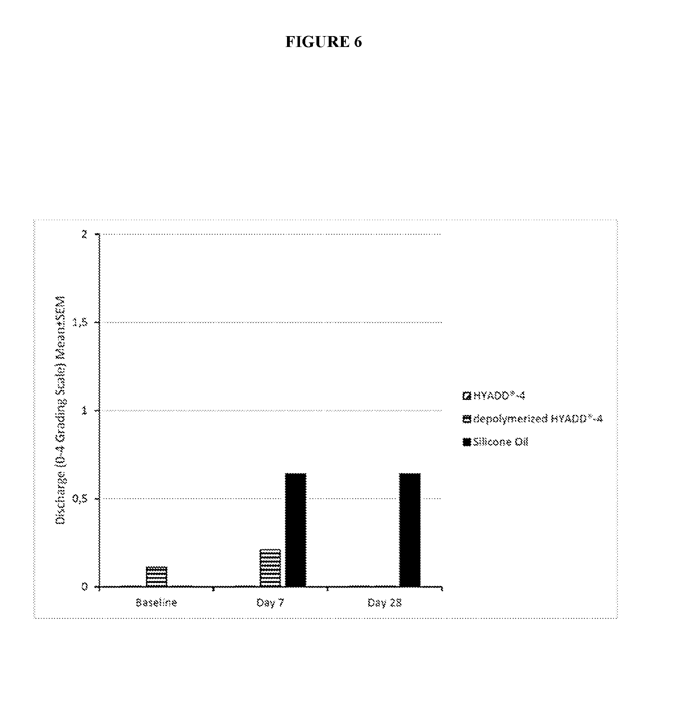

**FIG. 6****.** Evaluation of ocular discharge showed from rabbits in normal conditions (Baseline) and after 7 (Day 7) or 28 (Day 28) days from the surgery of retinal detachment induced by subretinal injection in the lower quadrant of the eye, with subsequent vitrectomy and complete replacement of the vitreous with silicone oil (Silicone oil), hexadecyl amide of hyaluronic acid (HYADD^{®}-4), or hexadecyl amide of hyaluronic acid depolymerized by heat treatment (depolymerized HYADD^{®}-4).

## Claims

1. Compositions comprising hexadecyl amide of hyaluronic acid with amidation degree comprised in the range 1-3% wherein the hexadecyl amide of hyaluronic acid is obtained by derivatization of hyaluronic acid of molecular weight MW comprised between 500,000 and 730,000 Da, for use in the surgical treatment of the eye.

2. Compositions for use according to claim 1 in the surgical treatment of the posterior segment of the eye, comprising the vitreous chamber.

3. Compositions for use according to claim 1 as biocompatible substitutes of the vitreous humor following vitrectomy.

4. Compositions for use according to claims 1-3 wherein the hexadecyl amide of hyaluronic acid is used as it is.

5. Compositions for use according to claims 1-3 wherein the hexadecyl amide of hyaluronic acid is depolymerized.

6. Compositions for use according to claim 5 wherein the hexadecyl amide of hyaluronic acid is depolymerized by thermic treatment at 121 °C for at least 25 minutes, preferably for 2 hours.

7. Compositions for use according to claims 1-6 in the form of hydrogel comprising the hexadecyl amide of hyaluronic acid in concentrations not higher than 12% by weight, preferably in concentrations of 8% by weight.

8. Compositions for use according to claims 1-7 wherein the surgical treatment is performed in case of severe traumas interesting the whole eyeball, infections of the internal part of the eye, diabetic retinopathy, retinal detachment, macular hole or formation of an epiretinal membrane in the central portion of the retina, vitreous hemorrhage, presence of floaters, or thickening, in the vitreous humor, complications of surgeries of the anterior segment of the eye, such as following cataract surgery.

## Patentansprüche

1. Zusammensetzungen, die Hyaluronsäurehexadecylamid mit einem Amidierungsgrad im Bereich von 1 bis 3 % enthalten, wobei das Hyaluronsäurehexadecylamid durch Derivatisierung von Hyaluronsäure mit einem Molekulargewicht MW zwischen 500.000 und 730.000 Da erhalten wird, zur Verwendung bei der chirurgischen Behandlung des Auges.

2. Zusammensetzungen zur Verwendung gemäß Anspruch 1 bei der chirurgischen Behandlung des hinteren Augenabschnitts, einschließlich der Glaskörperkammer.

3. Zusammensetzungen zur Verwendung gemäß Anspruch 1 als biokompatible Ersatzstoffe für den Glaskörper nach einer Vitrektomie.

4. Zusammensetzungen zur Verwendung gemäß den Ansprüchen 1-3, wobei das Hexadecylamid von Hyaluronsäure unverändert verwendet wird.

5. Zusammensetzungen zur Verwendung gemäß den Ansprüchen 1-3, wobei das Hexadecylamid von Hyaluronsäure depolymerisiert ist.

6. Zusammensetzungen zur Verwendung gemäß Anspruch 5, wobei das Hexadecylamid von Hyaluronsäure durch thermische Behandlung bei 121 °C für mindestens 25 Minuten, vorzugsweise für 2 Stunden, depolymerisiert wird.

7. Zusammensetzungen zur Verwendung gemäß den Ansprüchen 1 bis 6 in Form eines Hydrogels, das das Hexadecylamid von Hyaluronsäure in Konzentrationen von nicht mehr als 12 Gew.-%, vorzugsweise in Konzentrationen von 8 Gew.-%, enthält.

8. Zusammensetzungen zur Verwendung gemäß den Ansprüchen 1-7, wobei die chirurgische Behandlung bei schweren Traumata des gesamten Augapfels, Infektionen des Augeninneren, diabetischer Retinopathie, Netzhautablösung, einem Makulaloch oder der Bildung einer epiretinalen Membran im zentralen Bereich der Netzhaut, Glaskörperblutung, Vorhandensein von Floatern oder Verdickungen im Glaskörper, Komplikationen bei Operationen am vorderen Augenabschnitt, wie beispielsweise nach einer Kataraktoperation, durchgeführt wird.

## Revendications

1. Compositions comprenant de l'hexadécylamide d'acide hyaluronique avec un degré d'amidation compris dans la plage 1 à 3 %, dans lesquelles l'hexadécylamide d'acide hyaluronique est obtenu par dérivatisation d'acide hyaluronique de poids moléculaire MW compris entre 500 000 et 730 000 Da, pour une utilisation dans le traitement chirurgical de l'œil.

2. Compositions pour une utilisation selon la revendication 1 dans le traitement chirurgical du segment postérieur de l'œil, comprenant la chambre vitrée.

3. Compositions pour une utilisation selon la revendication 1 comme substituts biocompatibles de l'humeur vitrée suite à une vitrectomie.

4. Compositions pour une utilisation selon les revendications 1 à 3 dans lesquelles l'hexadécylamide d'acide hyaluronique est utilisé tel quel.

5. Compositions pour une utilisation selon les revendications 1 à 3 dans lesquelles l'hexadécylamide d'acide hyaluronique est dépolymérisé.

6. Compositions pour une utilisation selon la revendication 5, dans lesquelles l'hexadécylamide d'acide hyaluronique est dépolymérisé par traitement thermique à 121 °C pendant au moins 25 minutes, de préférence pendant 2 heures.

7. Compositions pour une utilisation selon les revendications 1 à 6 sous forme d'hydrogel comprenant l'hexadécylamide d'acide hyaluronique à des concentrations ne dépassant pas 12 % en poids, de préférence à des concentrations de 8 % en poids.

8. Compositions pour une utilisation selon les revendications 1 à 7 dans lesquelles le traitement chirurgical est effectué en cas de traumatismes graves touchant l'ensemble du globe oculaire, d'infections de la partie interne de l'œil, de rétinopathie diabétique, de décollement de rétine, de trou maculaire ou de formation d'une membrane épirétinienne dans la partie centrale de la rétine, d'hémorragie du corps vitré, de présence de corps flottants ou d'épaississement de l'humeur vitrée, de complications de chirurgies du segment antérieur de l'œil, comme suite à une chirurgie de la cataracte.
